# EUROPÄISCHE PATENTSCHRIFT

(11) **EP 1 107 993 B1**
(45) Veröffentlichungstag und Bekanntmachung des Hinweises auf die Patenterteilung: **25.10.2006**
(21) Anmeldenummer: 99944540.6
(22) Anmeldetag: 27.08.1999
(51) Int. Cl.: C07K 16/18, C12N 5/10, G01N 33/577, G01N 33/68, C07K 16/42

(54) **HUMANE MONOKLONALE ANTIKÖRPER GEGEN INSELZELLANTIGEN IA-2**
HUMAN MONOCLONAL ANTIBODIES DIRECTED AGAINST THE ISLET CELL ANTIGEN IA-2
ANTICORPS MONOCLONAUX HUMAINS CONTRE L'ANTIGENE DES CELLULES D'ILOTS DE LANGERHANS IA-2

(30) Priorität: 01.09.1998 DE 19839736
(43) Veröffentlichungstag der Anmeldung: 20.06.2001
(73) Patentinhaber: Roche Diagnostics GmbH, 68305 Mannheim (DE)
(72) Erfinder: ENDL, Josef, D-82362 Weilheim (DE); WILD, Thomas, D-82362 Weilheim (DE); BERLO, Suzanne, Elisabeth, NL-5076 GL Haaren (NL); LITTY, Verena, D-80336 München (DE)
(86) Internationale Anmeldenummer: PCT/EP1999/006321
(87) Internationale Veröffentlichungsnummer: WO 2000/012558

(56) Entgegenhaltungen:
- EP-A- 0 499 176
- US-A- 5 200 318
- M. SOLIMENA ET AL.: "ICA 512, an autoantigen of type I diabetes, is an intrinsic membrane protein of neurosecretory granules." THE EMBO JOURNAL, Bd. 15, Nr. 9, 1. Mai 1996 (1996-05-01), Seiten 2102-2114, XP002123338 Oxford, Grossbritannien in der Anmeldung erwähnt
- A. MADEC ET AL.: "Four IgG anti-islet human monoclonal antibodies isolated from a type 1 diabetes patient recognize distinct epitopes of glutamic acid decarboxylase 65 and are somatically mutated." THE JOURNAL OF IMMUNOLOGY, Bd. 156, Nr. 9, 1. Mai 1996 (1996-05-01), Seiten 3541-3549, XP002123339 Baltimore, MD, VSA in der Anmeldung erwähnt
- M. PAYTON ET AL.: "Relationship of the 37,000- and 40,000-Mr tryptic fragments of islet antigens in insulin-dependent diabetes to the protein tyrosine phosphatase-like molecule IA-2." THE JOURNAL OF CLINICAL INVESTIGATION, Bd. 96, 1. September 1995 (1995-09-01), Seiten 1506-1511, XP000612574 New York, NY, VSA in der Anmeldung erwähnt

## Beschreibung

Gegenstand der vorliegenden Erfindung sind humane monoklonale Antikörper gegen das Inselzellantigen IA-2, ein Verfahren zu ihrer Herstellung, die Verwendung der humanen monoklonalen Antikörper in einem Verfahren zum Nachweis von Antikörpern gegen IA-2, ein Verfahren zum Nachweis von Antikörpern gegen Inselzellantigen IA-2, sowie ein Verfahren zum Nachweis von IA-2.

Der insulinabhängige Diabetes mellitus vom Typ I (IDDM) beruht auf einer autoimmunen Zerstörung der insulinproduzierenden β-Zellen des Pankreas. Die Entwicklung von Autoantikörpern gegen β-Zellantigene geht dabei der Entwicklung eines klinisch diagnostizierbaren Diabetes voraus. Diese Autoantikörper dienen als sensitive Marker zur Identifikation der präklinischen Krankheitsphase. Auch in frisch diagnostizierten Diabetiker-Patienten findet man häufig Antikörper, die mit den β-Zellen in den Langerhans'schen Inseln reagieren. Die Gesamtheit der Autoantikörper wird auch als Islet Cell Antibodies (ICA) bezeichnet.

ICA sind sensitive und spezifische Marker für die Prognose und Diagnose von humanem IDDM. Zu den bisher charakterisierten Inselzellantigenen, gegen die Autoantikörper gebildet werden, zählen Insulin (Palmer et al. 1983, Science 222, 1337-1339), die Glutamat-Decarboxylase (GAD, Bækkeskov et al. 1990, Nature 347, 151-156), Carboxypeptidase H (Castano et al 1991, J. Clin. Endocrinol. Metab. 73, 1197-1201) Inselzellantigen ICA 69 (Pietropaolo et al. 1993, J. Clin. Invest. 92, 359-371) und das Antigen IA-2, das auch als ICA512 bezeichnet wird (Solimena et al. 1996, EMBO J. 15, 2102-2114).

Bislang konnte noch nicht geklärt werden, ob Autoantikörper, die gegen β-Zellantigene gerichtet sind, zur Entwicklung der Krankheit direkt beitragen oder ob das Auftreten der Autoantikörper ein Phänomen ist, das nach der Zerstörung der β-Zellen auftritt.

Nach heutigem Kenntnisstand ist jedoch das Auftreten von Autoantikörpern mit der Entwicklung eines Diabetes korreliert.

Es hat sich gezeigt, daß insbesondere Autoantikörper gegen IA-2 in den meisten frisch diagnostizierten IDDM-Patienten auftreten und daß die IA-2-spezifischen Autoantikörper mit einem schnellen Fortschreiten der Diabetes-Erkrankung assoziiert sind. IA-2-spezifische Autoantikörper scheinen darüberhinaus spezifischer für IDDM zu sein als GAD-Autoantikörper und außerdem weniger häufig bei anderen Autoimmunkrankheiten ohne IDDM aufzutreten (Roll und Ziegler 1997, Exp. Clin. Endocrinol. Diabetes 105, 1-14).

Beim Autoantigen IA-2 handelt es sich um ein Transmembran-Protein mit einem membran-durchquerenden Segment und einer cytoplasmatischen Domäne (IA-2ic), die die Epitope für die Antikörperbindung enthält (Solimena et al. 1996, EMBO J. 15, 2102-2114). Als intrinsisches Membran-Protein sekretorischer Vesikel wird IA-2 in peptidsekretierenden endokrinen Zellen sowie in Neuronen, die neurosekretorische Vesikel enthalten, exprimiert. IA-2 besitzt eine signifikante Homologie zu IA-2β, das auch unter der Bezeichnung Phogrin bekannt ist. IA-2β ist wie IA-2 ein Transmembranprotein, wird jedoch anders als IA-2 bevorzugt in β-Zellen exprimiert. IA-2β und IA-2 sind Proteine vom Rezeptortyp und gehören beide zur Klasse der Protein-Tyrosin-Phosphatasen (Roll und Ziegler 1997, supra).

Die Bestimmung der ICA (Islet Cell Antibodies) zum Nachweis von IDDM erfolgt im Stand der Technik durch Messungen an Pankreas-Gewebeschnitten mit indirekter Immunfluoreszenz. Hierbei werden die Autoantikörper in der zu untersuchenden Probe, die an die Inselzellstrukturen binden, durch fluoreszenzmarkierte, für Human-IgG spezifische Antikörper nachgewiesen. Allerdings sind diese ICA-Messungen technisch sehr aufwendig und schwierig zu standardisieren, da die Ergebnisse, die mit unterschiedlichem pankreatischen Gewebe von verschiedenen Spendern erhalten werden, stark variieren.

Im Stand der Technik werden Autoantikörper gegen IA-2 und GAD auch durch einfache Radioliganden-Bindungsassays in Serumproben bestimmt. Dabei werden in vitro translatierte, radioaktiv markierte Antigene verwendet (Dittler, J. et al 1998, Diabetes 47, 592-597). Zur Herstellung der radioaktiv markierten Antigene wird die cDNA des jeweiligen Antigens mittels eines Kaninchen-Retikulozyten-Lysates in vitro transkribiert. Die mRNA wird dann in Anwesenheit von radioaktiv markierten Aminosäuren (im allgemeinen mit ³⁵S, Schwefel-35 markiert) translatiert. Die Bindung der Autoantikörper an das markierte Antigen wird nach Abtrennung des Antigen-Antikörperkomplexes vom freien Antigen beispielsweise durch Filtration oder Festphasenbindung über das radioaktive Signal des markierten Antigens nachgewiesen.

Diese Nachweismethoden können zwar zum Teil automatisiert werden, haben jedoch den großen Nachteil, daß radioaktiv gearbeitet werden muß, was aufwendige und kostspielige Vorsichtsmaßnahmen erfordert. Die durch in-vitro-Translation erzielbaren Markierungseffizienzen für das Antigen sind von Batch zu Batch sehr variabel. Außerdem sind die markierten Antigene wegen eintretender Radiolyse bzw. wegen der kurzen Halbwertszeit des Schwefel-35 nur kurze Zeit haltbar.

Ein diagnostisch einfach, schnell und in automatisierter Form durchzuführender Test zum direkten Nachweis der IA-2-spezifischen Autoantikörper ist im Stand der Technik bisher nicht beschrieben. Dies ist insbesondere darauf zurückzuführen, daß bisher keine standardisierten IA-2-spezifischen Autoantikörper existieren. Als Standard- oder Eichmaterial wurden bisher lediglich hochtitrige Seren von IDDM-Patienten verwendet. Der Nachteil hierbei ist, daß ein solches Serum nicht in unbegrenzter Menge zur Verfügung steht und somit chargen- bzw. patientenabhängige Schwankungen im Antikörpergehalt des jeweiligen Standardserums bestehen. Ein Vergleich von Versuchsergebnissen aus unterschiedlichen Laboratorien ist dadurch nicht gegeben.

Aufgabe war es daher, humane monoklonale Antikörper zur Verfügung zu stellen, die spezifisch das Inselzellantigen IA-2 erkennen, sowie ein diagnostisches Testverfahren zum quantitativen Nachweis der IA-2-spezifischen Autoantikörper mittels einer Standardkurve zur Verfügung zu stellen, das die Nachteile des Standes der Technik zum großen Teil überwindet.

Die Aufgabe wird gelöst durch humane monoklonale Antikörper, die spezifisch mit dem Inselzellantigen IA-2 reagieren. Dazu gehören beispielsweise die Antikörper, die von der Zellinie IA-2, 96-3-1, hinterlegt am 13.08.1998 unter der Nummer DSM ACC2365 bei der DSMZ (Deutsche Sammlung von Mikroorganismen und Zellkulturen GmbH, Braunschweig, Deutschland), produziert werden.

Gegenstand der Erfindung sind daher humane monoklonale Antikörper gegen das Inselzellantigen IA-2 wie in Anspruch 1 definiert. Bevorzugt sind diese Antikörper vom IgG-Isotyp, besonders bevorzugt vom IgG1-Isotyp.

Im Stand der Technik ist bekannt, daß gegen das Inselzellantigen GAD (Glutamat-Decarboxylase) humane Autoantikörper erzeugt werden können (EP-A-0 499 176). Das dort beschriebene Verfahren umfaßt folgende Schritte:

Immortalisieren von humanen Lymphozyten von Prädiabetikern oder Diabetikern, Behandeln des Kulturüberstandes der (durch EBV-Transformation) immortalisierten Zellen mit einem Konjugat aus Antikörpern gegen humanes Fcγ und einer Markierung, anschließende Behandlung mit humanem Immunglobulin, Inkubieren mit immobilisierten humanen Pankreas-Inselzellen oder immobilisierter GAD, Identifizierung einer immortalisierten humanen Zellkultur, die einen Antikörper gegen Pankreas-Inseizellen produziert, über die Bestimmung der an die immobilisierten Inselzellen oder die immobilisierte GAD gebundene Markierung, Isolierung einer humanen immortalisierten Zelle, die diesen Antikörper produziert, Vermehrung dieser immortalisierten Zelle und Isolierung des von diesen Zellen produzierten monoklonalen Antikörpers.

Mit dem in EP-A-0 499 176 beschriebenen Verfahren ist es jedoch nicht möglich, humane monoklonale Antikörper gegen IA-2 herzustellen. Die erste Schwierigkeit zeigt sich bereits bei der Auswahl der Spender-Lymphozyten. Es können nicht beliebige Spender-Lymphozyten verwendet werden, sondern die Lymphozyten müssen von Prädiabetikern oder Diabetikern mit ausgewählt hohen IA-2-spezifischen Serumantikörper-Titern stammen.

Die Antikörper-Titer werden durch einen Radioliganden-Bindungsassay ermittelt. Bei diesem Assay wird die für IA-2 kodierende DNA mittels eines Retikulozyten-Lysates in vitro transkribiert und in Anwesenheit von ³⁵S-Methionin translatiert. Anschließend werden wenige Mikroliter (2 - 5 µl haben sich als geeignet erwiesen) Patientenserum mit dem radioaktiv markierten IA-2 inkubiert und die Immunkomplexe über Protein-A Sepharose vom freien Antigen abgetrennt. Die Bestimmung der an die Protein-A Sepharose gebundene Radioaktivität erfolgt in einem Szintillationszähler. Durch ein hochtitriges ausgewähltes Patientenserum werden die gemessenen cpm in arbiträre Units umdefiniert. Zum Beispiel wurde bei dem in dieser Studie verwendeten Patientenserum definiert, daß 1000 cpm 100 U entsprechen. Dieses Patientenserum wird bei Bestimmungen als arbiträrer Standard mitgeführt. Für die Transformationen wurden nur Lymphozyten verwendet, deren Spender einen Titer von größer als 80 U aufwiesen, da nur von diesen Spendern IA-2 positive Primärkulturen entdeckt wurden.

Darüberhinaus hat sich eine Vorselektion auf IgG-produzierende B-Lymphozyten als sinnvoll erweisen. Im peripheren Blut befinden sich ca. 10mal mehr IgM-produzierende B-Lymphozyten als IgG-produzierende B-Zellen. Andererseits sind die relevanten Autoantikörper gegen IA-2 vom IgG-Subtyp (Zhang et al 1997, Diabetes 46, 40-43). Durch Isolierung der Membran-IgG positiven B-Lymphozyten kann die relevante B-Zell Subpopulation um einen Faktor 10 angereichert werden. Die Isolierung erfolgt durch Markierung der humanen B-Lymphozyten mit einem für Human-IgG spezifischen Antikörper aus der Maus und nachfolgender Bindung von Magnetobeads, die mit Schaf-anti-Maus-IgG beladen sind. Durch Anlegen eines Magneten können die markierten Zellen aus der Zellsuspension positiv selektioniert werden.

Weiterhin zeigen sich Probleme bei der Anzucht der immortalisierten Lymphozyten, da die immortalisierten IA-2 spezifischen B-Zellen nur eine geringe Proliferationsrate aufweisen und häufig von unspezifischen, aber schnell wachsenden immortalisierten B-Zellen überwachsen werden. Es hat sich gezeigt, daß durch den Zusatz von Wachstumsfaktoren wie IL-6 oder IL-10 die Proliferation der immortalisierten IA-2-spezifischen B-Zellinien entscheidend verbessert werden kann. Ein zusätzliches Problem war, daß die immortalisierten B-Zellinien Faktoren sezernierten, die das Wachstum der Zellinien negativ beeinflussen. Zu diesen Faktoren gehören vor allem TGF-beta, IF-gamma und TNF-alpha. Die Entfernung dieser inhibitorischen Faktoren durch häufiges Wechseln des Kulturmediums bewirkt eine höhere Transformationsrate und ein schnelleres Wachstum der EBV transformierten B-Zell Linien.

Entscheidend für die erfolgreiche Klonierung (siehe unten) der entstehenden EBV-transformierten B-Lymphozyten ist es, von vornherein limitierte Mengen an B-Lymphozyten in die einzelnen Wells (Vertiefungen der Mikrotiterplatte) einzubringen. Im Stand der Technik werden mehrere tausend gereinigte periphere mononukleäre Zellen pro Well empfohlen (Peyron, E. et al 1994, J. Immunology 153, 1333-1339; Madec, A.-M. et al 1996, J. Immunology 156, 3541-3549). Überraschenderweise hat sich gezeigt, daß für die Primärtransformation nur maximal 400 IgG-positive Zellen/Well eingesetzt werden können. Die Transformationsrate beträgt etwa 1 von 80 B-Zellen, das heißt, pro Well wachsen maximal 5 verschiedene Klone. Dadurch erhöht sich die Wahrscheinlichkeit, daß man bei der anschließenden Einzelzell-Klonierung, bei der nur wenige EBV-transformierte B-Zell Klone wachsen (oft nur 1-2%), die relevanten Klone isolieren kann. Ebenfalls überraschend ist, daß sich die meisten positiven Primärwells bei noch niedrigerer Einsaatdichte isolieren ließen, was dahingehend interpretiert werden kann, daß bereits bei 400 B-Zellen pro Well zuviele Klone entstehen und dadurch langsamer wachsende Klone von schneller wachsenden unterdrückt werden.

Nach einer Wachstumsperiode von ca. 2 Wochen werden die Kulturüberstände der Primär-Zellkulturen dann beispielsweise mittels eines ELISA-Tests an immobilisiertem IA-2 auf die Produktion von IA-2-spezifischen Antikörpern getestet. Besonders geeignet hat sich erfindungsgemäß das Screening auf IA-2-spezifische Antikörper mittels der cytoplasmatischen Domäne des IA-2 erwiesen, die auch als IA-2ic bezeichnet wird.

Bei der anschließenden Einzelzell-Klonierung zur Stabilisierung der Zellinien müssen sogenannte Feederzellen zugesetzt werden, da EBV-transformierte B-Zellinien in niedriger Zelldichte (<25 pro Well) nicht überlebensfähig sind. Als Feederzellen dienen autologe (vom selben Spender stammende) oder allogene (von einem anderen Spender stammende), mit 4000 rad bestrahlte periphere Blutlymphozyten.

Die Klonierungseffizienz kann dadurch entscheidend verbessert werden, daß man die cytotoxischen T-Lymphozyten (CD8 positive Zellen) aus der Feederzell-Population entfernt. Die Entfernung der CD8-Zellen erfolgt bevorzugt durch immunomagnetische Separation. Dazu werden die peripheren Blutlymphozyten zum Beispiel mit magnetischen Microbeads inkubiert, an die monoklonale Antikörper gegen das humane CD8 Antigen gekoppelt sind. Durch Anlegen eines Magneten werden die markierten Zellen entfernt, die restlichen Zellen bestrahlt und in einer Konzentration von 20 000 - 50 000 Zellen pro Well eingesetzt.

Ebenfalls als vorteilhaft bei der Herstellung der erfindungsgemäßen Antikörper hat es sich erwiesen, eine Qualitätsüberprüfung der Feederzellen durchzuführen. Es konnte festgestellt werden, daß sich die wachstumsunterstützende Funktion dieser Feederzellen von Spender zu Spender stark unterscheidet. Die Feederzellen von manchen Spendern wirkten sogar inhibierend auf das Wachstum. Deshalb war ein wichtiger Fortschritt dadurch erzielbar, daß jeder neue Batch an Feederzellen zunächst auf einer etablierten monoklonalen EBV-transformierten B-Zellinie (MICA 5) als Testzellinie auf seine Eignung für die Klonierungsprozedur untersucht wurde und "schädliche" Feederbatches aussortiert wurden. Dazu wurden Einzelzell-Klonierungen von MICA 5 auf bestrahlten Blutlymphozyten von verschiedenen Spendern durchgeführt. Nach ca. 3 Wochen wurde die Klonierungseffizienz durch mikroskopische Bestimmung der Anzahl der bewachsenen Wells bestimmt. Es wurden nur solche Feederzellen verwendet, mit denen eine Klonierungseffizienz von mindestens 20% erzielt werden konnte.

Der Immortalisierungsschritt kann durch die dem Fachmann bekannte Transformation mit EBV (Epstein-Barr-Virus) erfolgen. Diese Transformationsmethode ist erfindungsgemäß bevorzugt. Die am häufigsten in der Literatur beschriebene Methode zur EBV-Transformation (siehe Ifversen, P. et al 1993, Hum. Antibod. Hybridomas 4, 115-123) besteht darin, die B-Lymphozyten mit EBV für 2-3 Stunden zu inkubieren, um die Virusaufnahme zu erlauben. Danach werden die Zellen gewaschen und dadurch das Virus entfernt. Überraschenderweise konnte jedoch festgestellt werden, daß eine erhöhte Transformationsrate dadurch zu erzielen ist, daß man das Virus nicht auswäscht, sondern während der gesamten Inkubationsdauer bis zum ersten Medienwechsel (nach ca. 7 Tagen) zusammen mit den B-Zellen inkubiert.

Die Immortalisierung kann jedoch auch durch Fusion mit geeigneten Myeloma-Zellen durchgeführt werden. Denkbar ist auch die Herstellung der erfindungsgemäßen monoklonalen Antikörper gegen IA-2 über die sogenannte Phage-Display-Methode (Nissim, A. et al 1994, EMBO J. 13, 3, 692-698). Dabei wird mRNA direkt aus den Lymphozyten der IDDM-Patienten isoliert. Aus der damit hergestellten cDNA können die Immunglobulin-Gene amplifiziert werden (beispielsweise mittels Polymerasekettenreaktion). Die so erzeugten Gene können wiederum in einer Phagen-Library als Fab oder single chain Fv exprimiert werden, aus der dann die an IA-2 bindenden Phagen isoliert werden.

Erst durch die Überwindung der oben geschilderten Schwierigkeiten war es möglich, humane monoklonale Antikörper gegen das Inselzellantigen IA-2 herzustellen.

Ein bevorzugter Gegenstand der Erfindung sind daher monoklonale Antikörper, die von der Zellinie IA-2, 96-3-1, hinterlegt am 13.08.1998 unter der Nummer DSM ACC2365 bei der DSMZ (Deutsche Sammlung von Mikroorganismen und Zellkulturen GmbH, Braunschweig, Deutschland), produziert werden. Die Zellinie DSM ACC2365 ist ebenfalls Gegenstand der Erfindung.

Ebenfalls ein Gegenstand der Erfindung sind Antikörper, die in äquivalenter Weise wie die von der Zellinie IA-2, 96-3-1 (DSM ACC2365) produzierten mit IA-2 bindefähig sind. Unter dem Begriff "in äquivalenter Weise bindefähig" werden Antikörper verstanden, bei denen eine Epitopüberlappung mit dem definierten, bekannten Antikörper nachweisbar ist. Diese Epitopüberlappung kann mit Hilfe eines kompetitiven Testsystems leicht nachgewiesen werden. Dazu wird zum Beispiel mit Hilfe eines Enzym-Immunoassays überprüft, inwieweit ein Antikörper mit dem bekannten Antikörper um die Bindung an ein definiertes Antigen oder ein definiertes Epitop konkurriert (beispielsweise IA-2ic). Dazu inkubiert man beispielsweise immobilisiertes IA-2ic-Antigen mit dem bekannten monoklonalen Antikörper, der eine Markierung trägt und einem Überschuß des in Betracht gezogenen Antikörpers. Durch Nachweis der gebundenen Markierung kann dann leicht festgestellt werden, inwieweit der in Betracht gezogene Antikörper den definierten Antikörper aus der Bindung an das Antigen verdrängen kann. Ist eine Verdrängung von mindestens 50 % bei 10⁵-fachem Überschuß gegeben, so liegt eine Epitopüberlappung vor.

Ein weiterer Gegenstand der Erfindung sind außerdem humane monoklonale Antikörper gegen das Inselzellantigen IA-2, die erhältlich sind durch die Verfahrensschritte Immortalisieren von humanen Lymphozyten von Prädiabetikern oder Diabetikern mit hohen Serumantikörper-Titern (>80 U) gegen IA-2, Kultivierung der immortalisierten Lymphozyten mit Wachstumsfaktoren unter gleichzeitiger Entfernung von inhibitorischen Faktoren durch häufigen Medienwechsel, Nachweis der IA-2-spezifischen humanen monoklonalen Antikörper im Kulturüberstand bevorzugt mittels ELISA, Klonierung der humanen immortalisierten Zellinie, die diesen Antikörper produziert, in Anwesenheit von Feederzellen, die keine cytotoxischen T-Lymphozyten enthalten, Vermehrung dieser immortalisierten Zelle gegebenenfalls unter Zusatz von Wachstumsfaktoren, und Isolierung des von diesem Klon produzierten monoklonalen Antikörpers.

Ebenfalls ein Gegenstand der Erfindung ist ein Verfahren zur Herstellung von humanen monoklonalen Antikörpern, die spezifisch mit dem Inselzellantigen IA-2 reagieren, enthaltend die Schritte Immortalisieren von humanen Lymphozyten von Prädiabetikern oder Diabetikern mit hohen Serumantikörper-Titern (>80 U/ml) gegen IA-2, Kultivierung der immortalisierten Lymphozyten mit Wachstumsfaktoren unter gleichzeitiger Entfernung von inhibitorischen Faktoren durch häufigen Medienwechsel, Nachweis der IA-2-spezifischen humanen monoklonalen Antikörper im Kulturüberstand, bevorzugt mittels ELISA, Klonierung der humanen immortalisierten Zellinie, die diesen Antikörper produziert, in Anwesenheit von Feederzellen, die keine cytotoxischen T-Lymphozyten enthalten, Vermehrung dieser immortalisierten Zelle gegebenenfalls unter Zusatz von Wachstumsfaktoren, und Isolierung des von diesem Klon produzierten monoklonalen Antikörpers.

Die Durchführung der einzelnen Verfahrensschritte erfolgt so wie in den vorhergehenden Abschnitten beschrieben.

Unter dem Begriff "monoklonaler Antikörper" sind im Sinne der Erfindung neben den intakten Immunglobulinen auch sämtliche Antikörperfragmente zu verstehen. Dazu gehören beispielsweise Fab, Fab' oder F(ab)'₂-Fragmente. Wird der Begriff "Antikörper" ohne den Zusatz "monoklonal" oder "polyklonal" verwendet, so sind immer beide Arten von Antikörpern, sowie chimäre Konstrukte und alle oben aufgeführten Fragmente gemeint.

Die erfindungsgemäßen IA-2-spezifischen monoklonalen Antikörper reagieren spezifisch mit IA-2, wobei sie bevorzugt mit dem cytoplasmatischen Teil von IA-2, dem sogenannten IA-2ic reagieren. Gegenstand der Erfindung sind daher auch Antikörper gegen IA-2, die spezifisch mit dem cytoplasmatischen Teil von IA-2, dem sogenannten IA-2ic reagieren. Sie werden analog zu den zuvor beschriebenen Verfahrensschritten hergestellt.

Zur Identifikation der IA-2-spezifischen monoklonalen Antikörper wird bevorzugt ein ELISA-Test durchgeführt. Für das Auffinden von anti-IA-2 spezifischen EBV-transformierten B-Zellinien müssen je Spender mindestens 1000 Primärwells getestet werden. Ein solch umfangreiches Screening ist mit dem sehr arbeitsaufwendigen RIA des Standes der Technik nicht möglich. Der sehr hohe Probendurchsatz ist nur durch Entwicklung eines ELISA zu erzielen. Mit diesem halbautomatischen ELISA ist es möglich, mehrere tausend Kulturüberstände pro Tag zu testen und damit das sehr seltene Ereignis eines IA-2-positiven Primärwells zu entdecken .

Im ELISA werden mit Streptavidin beschichtete Mikrotiterplatten mit IA-2-Biotin oder IA-2ic-Biotin beschichtet. Anschließend werden die beschichteten Platten mit verschiedenen Verdünnungsstufen von Humanseren aus Prädiabetikern oder etablierten Diabetikern inkubiert. Parallel dazu werden auf derselben Mikrotiterplatte definierte Mengen eines gereinigten humanen IA-2-spezifischen Antikörpers inkubiert. Anschließend werden die Platten gewaschen und zum Nachweis von gebundenen Anti-IA-2-Antikörpern ein Peroxidase-markiertes Schaf-anti-Human-Fcγ-spezifisches Antikörperkonjugat zugegeben. Gebundene IA-2-spezifische Antikörper werden durch eine Farbreaktion mittels ABTS^{®} (Azino-di-[3-ethylbenzthiazolinsulfonat (6)], Kat. Nr. 756 407, Boehringer Mannheim GmbH Deutschland) nachgewiesen. Aus den Extinktionen der Standardkurve kann unter Berücksichtigung des Verdünnungsfaktors auf den Gehalt der Anti-IA-2-Antikörper in den Patientenseren geschlossen werden.

Zur Herstellung des Antigens IA-2ic, das im ELISA eingesetzt wird, wurde aus einer Inselzell-spezifischen cDNA das IA-2ic-Gen durch die folgenden Primer amplifiziert, die von Payton et al. (1995) in J. Clin. Invest. 96, 1506-1511 publiziert sind:
5'-ATGCAGCAAGACAACGAGCGCCTG-3' und
5'-TCACTGGGGCAGGGCCTTGAG-3'

Die Amplifikationsprodukte wurden in einen Pin Point Vektor kloniert und in E. coli als lösliches, biotinyliertes Fusionsprotein exprimiert. Das Fusionsprotein wurde an monomerer Avidin-Sepharose aufgereinigt. Das biotinylierte IA-2ic wurde an mit Streptavidin beladene Mikrotiterplatten gebunden, mit den erfindungsgemäßen IA-2-spezifischen monoklonalen Antikörpern inkubiert und gebundener Antikörper durch ein mit Peroxidase markiertes Anti-Human-IgG-Konjugat nachgewiesen. Um auszuschließen, daß die Antikörper gegen die biotinylierte aminoterminale Domäne des Fusionsproteins gerichtet waren, wurde auch die biotinbindende Domäne des Fusionsproteins (Tag-Protein) alleine exprimiert und im ELISA getestet. Die IA-2-spezifischen Antikörper erkannten das Tag-Protein nicht. Zusätzlich wurden die IA-2-spezifischen Antikörper in einem RIA getestet. Hierzu wurde die DNA für IA-2ic in vitro in Anwesenheit von ³⁵S-Methionin transkribiert und translatiert, mit den IA-2-spezifischen Antikörpern inkubiert und die Immunkomplexe durch Zugabe von Protein A-Sepharose immobilisiert. Die Radioaktivität in den Immunpräzipitaten wurde durch Flüssigszintillationszählung bestimmt.

Ebenfalls ein Bestandteil der vorliegenden Erfindung ist ein Verfahren zum Nachweis von humanen Antikörpern oder Autoantikörpern gegen das Inselzellantigen IA-2 in einer Probe. Als Testformate kommen alle dem Fachmann geläufigen Formate in Frage, wobei der indirekte ELISA-Test bevorzugt ist. Als geeignet hat es sich erwiesen, aufgereinigtes natives oder rekombinant hergestelltes IA-2-Antigen oder IA-2ic-Antigen mit der Probe in Kontakt zu bringen, so daß die Probenantikörper an das Antigen spezifisch binden können. Ist das Antigen mit einer festphasenbindefähigen Gruppe versehen wie beispielsweise Biotin, so kann anschließend die Immobilisierung des Immunkomplexes an einer mit Streptavidin beschichteten Festphase erfolgen. Das Antigen kann auch bereits direkt oder indirekt an der Festphase gebunden sein, wenn die Inkubation mit der Probe stattfindet. Der Nachweis der Probenantikörper erfolgt nach Trennung der festen von der flüssigen Phase bevorzugt durch Bindung eines mit einer Markierung versehenen Antikörpers, der gegen den Fc-Teil von humanen Antikörpern, im allgemeinen den Fc-Teil von humanem IgG gerichtet ist, und anschließende Messung der Markierung. Als Markierung können alle dem Fachmann geläufigen Markierungen verwendet werden, wie beispielsweise Enzyme wie Peroxidase, Haptene wie Digoxigenin, Fluoreszenzfarbstoffe oder zur Elektrochemilumineszenz oder Chemilumineszenz fähige Substanzen.

Denkbar ist auch ein kompetitives Testformat, bei dem das IA-2- oder IA-2ic-Antigen direkt oder indirekt an einer Festphase gebunden ist und ein erfindungsgemäßer humaner monoklonaler Antikörper gegen IA-2 oder IA-2ic, der eine Markierung trägt, in einer definierten Konzentration als Rezeptor zugesetzt und mit dem Antigen inkubiert wird. Wird die Probe gleichzeitig oder danach hinzugefügt, so kompetieren die Probenantikörper und der markierte Rezeptorantikörper miteinander um die Bindung an das Antigen. Nach Trennung der festen von der flüssigen Phase wird die Markierung in einer oder beiden Phasen bestimmt. Ein geringes Signal der Markierung an der Festphase deutet auf eine hohe Konzentration an Probenantikörper hin.

Durch Vergleich der erhaltenen Meßwerte für die Proben mit Meßwerten, die mittels einer Standardreihe zuvor ermittelt werden, kann eine Quantifizierung der Probenantikörper erfolgen. In einer solchen Standardreihe werden definierte Konzentrationen der erfindungsgemäßen monoklonalen Antikörper gegen IA-2 oder IA-2ic eingesetzt.

Als Proben für den Nachweis von Antikörpern gegen IA-2 können alle dem Fachmann geläufigen Körperflüssigkeiten verwendet werden. Dazu zählen beispielsweise Vollblut, Serum oder Plasma, Urin und Speichel.

Ein weiterer Gegenstand der Erfindung ist die Verwendung eines humanen monoklonalen Antikörpers gegen IA-2 oder IA-2ic als Standard oder als Rezeptor in einem Verfahren zur Bestimmung von Antikörpern gegen ein Inselzellantigen, bevorzugt gegen das Inselzellantigen IA-2.

Ein weiterer Gegenstand der Erfindung ist die Verwendung eines humanen monoklonalen Antikörpers gegen das Inselzellantigen IA-2 zur Gewinnung des Inselzellantigens IA-2. Zur Gewinnung des Inselzellantigens IA-2 können die erfindungsgemäßen Antikörper nach dem Fachmann bekannten Verfahren an eine Festphase gekoppelt werden.

Anschließend wird die IA-2-haltige Probe mit den Festphasen-gebundenen Antikörpern inkubiert und die übrigen Bestandteile abgetrennt. Durch Spaltung des Immunkomplexes zwischen Antikörper und Antigen beispielsweise durch eine hohe Salzkonzentration und anschließender Elution kann das Antigen in reiner Form erhalten werden.

Gegenstand der Erfindung sind auch anti-idiotypische Antikörper, deren Antigenbindungsstelle der Struktur des Antigens IA-2 bzw. IA-2ic entspricht. Erhältlich ist ein solcher anti-idiotypischer Antikörper durch Immunisierung mit einem erfindungsgemäßen humanen Antikörper gegen IA-2, Immortalisierung der Milzzellen der immunisierten Tiere, Klonierung von solchen immortalisierten Zellen, die Antikörper produzieren, die an die Bindungsregion der IA-2 spezifischen Antikörper binden und Isolierung der von diesen Klonen produzierten Antikörper nach bekannten Verfahren.

Ebenfalls ein Gegenstand der Erfindung ist ein Verfahren zum Nachweis von IA-2 in einer Probe, das dadurch gekennzeichnet ist, daß als Bindepartner mindestens ein erfindungsgemäßer monoklonaler Antikörper dazu eingesetzt wird. Bevorzugt wird der Nachweis als Sandwich-ELISA durchgeführt. Dabei wird als ein Bindepartner ein Antikörper gegen IA-2, der ein erfindungsgemäßer Antikörper sein kann, nach dem Fachmann bekannten Methoden (beispielsweise über Biotin/Streptavidin) an eine Festphase gebunden. Das in der Probe vorhandene IA-2 bindet an den festphasengebundenen Antikörper. Der Nachweis des gebundenen IA-2 erfolgt über einen weiteren Bindpartner, der eine Markierung trägt. Der weitere Bindepartner ist ebenfalls bevorzugt ein Antikörper und bindet ebenfalls spezifisch an IA-2, erkennt jedoch ein anderes Epitop als der festphasengebundene Bindepartner. Der markierte Bindepartner kann ein erfindungsgemäßer monoklonaler Antikörper sein, wenn der festphasengebundene Antikörper ein anderes Epitop erkennt. Als Markierung können alle dem Fachmann geläufigen Markierungen eingesetzt werden. Dazu zählen beispielsweise Enzyme wie Peroxidase, Haptene wie Digoxigenin, Fluoreszenzfarbstoffe, zur Elektro- oder Chemilumineszenz fähige Substanzen.

Die folgenden Beispiele erläutern die Erfindung weiter.

### Beispiel 1:

### Auswahl der Spender für die Isolierung von B-Lymphozyten

Um die Wahrscheinlichkeit für eine erfolgreiche Transformation von anti-IA-2-spezifischen B-Lymphozyten aus dem peripheren Blut zu erhöhen, wurden für die Lymphozyten-Isolierung Spender ausgewählt, die einen hohen Serum-Antikörper Titer gegen IA-2 aufwiesen.

Die Antikörper wurden dabei durch einen in vitro Translationsassay bestimmt (siehe Zhang et al 1997, Diabetes 46, 40-43 sowie Dittler J. et al 1998, Diabetes 47, 592-597). Frisch diagnostizierten Diabetikern wurde Blut entnommen und nach bekanntem Verfahren Serum gewonnen. Ein Volumen von 2 - 5 µl der einzelnen Seren wurde in 50 µl Präzipitationspuffer (20 mM Tris-HCl, pH 7,5 , 150 mM NaCl, 1% Triton X-100, 0,1% Aprotinin) mit dem durch in vitro Translation radioaktiv markierten IA-2ic Polypeptid (ca. 15 000 cpm) über Nacht bei 4°C unter Rotation inkubiert. Anschließend wurden 50 µl einer 50%igen Protein A Sepharose-Suspension zugegeben und für eine weitere Stunde inkubiert. Danach wurde dreimal mit dem Inkubationspuffer gewaschen und die Radioaktivität der Beads in einem Flüssigszintillationsgerät bestimmt. Als arbiträrer Standard wurde ein hochtitiriges Diabetiker-Serum verwendet, welches bei einer Serummenge von 5µl ca.1000 cpm im Immunopräzipitat aufwies. Dieser Wert wurde als gleichbedeutend mit 100 U definiert. Die Normalseren lagen damit bei ca. 5 U. Für die nachfolgenden Transformationen der peripheren Blutlymphozyten wurden nur Lymphozyten von Patienten verwendet, deren Seren einen Titer von größer als 80 U aufwiesen.

### Beispiel 2:

### Zellseparation und EBV-Transformation

Nur solche Lymphozytenspender, deren Seren mindestens einen Titer von 80 U aufwiesen, wurden für die Isolierung von B-Lymphozyten verwendet. Von diesen Spendern wurden 20-50 ml Blut abgenommen und daraus die peripheren mononukleären Zellen (PBMNC) über eine Dichtegradienten-Zentrifugation isoliert.

Die klassischen Tests zum Nachweis der IA-2-spezifischen Serum-Antikörper verwenden zur Abtrennung der Immunkomplexe Protein A-Sepharose. Deshalb muß angenommen werden, daß die anti-IA-2 Antikörper fast ausschließlich der IgG-Immunglobulinklasse angehören (Zhang et al, Diabetes, 1997, 46, 40-43 sowie Dittler J. et al, Diabetes, 1998, 47, 592-597). Da die B-Lymphozyten des peripheren Blutes jedoch überwiegend IgM produzieren, wurden zur Anreicherung der relevanten B-Zell Subpopulation die Membran-IgG positiven B-Zellen isoliert. Hierzu wurden die PBMNC mit eiskaltem IMDM/10% fötalem Kälberserum (IMDM/10% FKS) auf eine Konzentration von 3*10⁶ Zellen/ml eingestellt. Anschließend wurde ein Anti-Human-IgG-Antikörper aus der Maus in einer Konzentration von 10 µg/ml zugegeben. Die Zellen wurden dann 30 Minuten bei 4°C gerollert, um ein Absetzen der Zellen während der Inkubation zu vermeiden. Anschließend wurde bei 200*g für 10 Minuten bei Raumtemperatur zentrifugiert, der Überstand abgesaugt und die Zellen zweimal mit IMDM/10% FKS gewaschen.

Danach wurden die Zellen in IMDM/10% FKS in einer Konzentration von 1*10⁷ Zellen/ml (Gesamtzellzahl ca. 1*10⁷ Zellen) aufgenommen und mit Magnetobeads (Dynal M-280), die mit Schaf-Anti-Maus-IgG beladen waren, inkubiert. Es wurden etwa 10 Beads pro Zielzelle zugegeben (es wurde davon ausgegangen, daß etwa 5% der PBMNC Membran-IgG exprimieren).

Die Zellen wurden mit den Beads für 30 Minuten bei 4°C gerollert. Danach wurde das Reaktionsgefäß für 5 Minuten in den Magnethalter gestellt, um die markierten Zellen abzutrennen. Der Überstand wurde abgesaugt, die Beads in 1 ml Medium resuspendiert und nochmals für 5 Minuten in den Magnethalter gestellt. Der Überstand wurde erneut abgesaugt, das Röhrchen aus dem Magnethalter genommen und die isolierten Zellen in 0,5 ml IMDM/10% FKS resuspendiert.

Anschließend wurden 2 ml konzentrierte Epstein-Barr-Virus-Suspension zugeben. Die Virussuspension wurde aus dem Überstand einer konfluenten Kultur der B 95-8 Marmoset Zellinie (ATCC CRL 1612) gewonnen. Zur Virusadsorption wurden die B-Zellen für 2 Stunden im Brutschrank bei 37 °C, 7 % CO₂ inkubiert. Das Röhrchen wurde während der Inkubationsphase mehrmals bewegt, um ein Absetzen der Zellen zu vermeiden.

Danach wurde mit den separierten B-Zellen eine Verdünnungsreihe gemacht, sodaß 100, 200 und 400 B-Zellen in 100 µl IMDM/10% FKS enthalten waren. Diese Zellsuspensionen wurde dann mit 4000 rad bestrahlten allogenen PBMNC (ohne CD8-positive Zellen) als Feederzellen versetzt (50 000 Feederzellen pro 150 µl Zellsuspension). Anschließend erfolgte die Zugabe von 100 U/ml IL-6. Pro Well wurden 150 µl Zellsuspension in 96 Well Rundbodenplatten verteilt und für 2 Wochen bei 5% CO₂ und 37°C inkubiert. Nach 7-10 Tagen wurde das Medium gewechselt.

### Beispiel 3:

### Screening-Assay für IA-2-Antikörper produzierende EBV-transformierte B-Zell-Linien

Nach zwei Wochen wurden die Kulturüberstände der EBV-Linien in einem ELISA auf Reaktivität gegen rekombinantes IA-2 getestet. Als Antigen wurde der intrazelluläre Teil des IA-2 (IA-2ic) in Escherichia coli in Kombination mit einem Biotin-markierten Peptid am NH₂-Terminus exprimiert. Das Fusionsprotein wurde durch Affinitätschromatographie an einer Streptavidin-Säule aufgereinigt.

Mit Streptavidin beschichtete Mikrotiterplatten wurden mit IA-2ic-Biotin in einer Konzentration von 100 ng/ml für eine Stunde bei Raumtemperatur beschichtet. Anschließend wurden die Platten mit 0,15 mol/l NaCl/0,05 % Tween 20 gewaschen. In die Platten wurden 50µl RPMI/10%FKS vorgelegt und danach 50 µl Kulturüberstand der EBV-transformierten B-Zellen zugegeben. Es wurde 1 Stunde bei Raumtemperatur unter Schütteln inkubiert. Anschließend wurden die Platten gewaschen und zum Nachweis von gebundenen Anti-IA-2-Antikörpern 100µl eines Peroxidase-markierten Schaf-Anti-Human-Fcγ-Antikörpers (Boehringer Mannheim GmbH, Kat. Nr. 1089 196, 100 mU/ml in PBS/0,5% Rinderserumalbumin) zugegeben. Anschließend wurde wiederum für 1 Stunde bei Raumtemperatur unter Schütteln inkubiert. Überschüssiges Konjugat wurde durch dreimaliges Waschen mit 0,15 mol/l NaCl/0,05 % Tween 20 entfernt. Danach wurden 100 µl ABTS^{®} (1 mg/ml, Boehringer Mannheim GmbH, Kat. Nr. 756 407) in 40 mmol/l Citrat-Puffer pH 4,4, der 3,25 mmol/l Natriumperborat enthält, zugegeben und nach 45 minütiger Inkubation bei Raumtemperatur unter Schütteln die Extinktion bei 405 nm gemessen.

Bei einem typischen Transformationsansatz wurden aus anfänglich 1*10⁷ PBMNC 2-5*10⁵ Membran-IgG positive B-Zellen isoliert. Diese wurden auf ca. 1000 Näpfe verteilt. Die Zahl der im Screeningtest als positiv identifizierten Näpfe lag im Bereich zwischen 1-3 Näpfe pro 1000 getesteter Näpfe. Die Extinktion der positiven Näpfe lag im Bereich von 1500-2000 mE.

### Beispiel 4:

### Klonierung der EBV-transformierten B-Zell Linien

Diejenigen EBV-transformierten B-Zell Linien, deren Kulturüberstand eine positive Reaktion im ELISA ergab, wurden kloniert. Die Zellen wurden dazu mit Hilfe eines Fluoreszenz-aktivierten Zellsorters einzeln in 96er Mikrotiterplatten abgelegt und mit bestrahlten CD8-depletierten PBMNC (5*10⁴ Zellen/Vertiefung, 4000 rad) versetzt. Das Klonierungsmedium bestand aus IMDM/10%FKS/100 U/ml IL-6. Die Kulturüberstände mit wachsenden Klonen wurden mittels ELISA getestet und die positiven Klone expandiert. Die Massenkultur zur Isolierung von Antiköpern erfolgte in einem Tecnomaus-Bioreaktor. Die Antikörper wurden aus dem Überstand durch Ammoniumsulfatfällung und Chromatographie über Protein A- oder Protein-G-Sepharose isoliert.

## Patentansprüche

1. Humane monoklonale Antikörper, erhältlich durch die Verfahrensschritte Immortalisieren von humanen Lymphozyten von Prädiabetikern oder Diabetikern mit hohen Serumantikörper-Titern gegen IA-2, Kultivierung der immortalisierten Lymphozyten mit Wachstumsfaktoren unter gleichzeitiger Entfernung von inhibitorischen Faktoren, Nachweis der LA-2-spezifischen humanen monoklonalen Antikörper im Kulturüberstand, Klonierung der humanen immortalisierten Zellinie, die diesen Antikörper produziert, in Anwesenheit von Feederzellen, die keine cytotoxischen T-Lymphozyten enthalten, Vermehrung dieser immortalisierten Zelle gegebenenfalls unter Zusatz von Wachstumsfaktoren und Isolierung des von diesem Klon produzierten monoklonalen Antikörpers.

2. Humane monoklonale Antikörper gemäß Anspruch 1, **dadurch gekennzeichnet, daß** sie spezifisch mit IA-2ic, dem cytoplasmatischen Teil von IA-2 reagieren.

3. Humane monoklonale Antikörper nach einem der Ansprüche 1 bis 2, **dadurch gekennzeichnet, daß** sie der Klasse IgG angehören.

4. Humane monoklonale Antikörper nach Anspruch 3, **dadurch gekennzeichnet, daß** sie der Subklasse IgG1 angehören.

5. Humane monoklonale Antikörper nach den Ansprüchen 1 bis 4, erhältlich aus der Zellinie IA-2, 96-3-1 mit der Hinterlegungsnummer DSM ACC2365.

6. Humane monoklonale Antikörper nach den Ansprüchen 1 bis 5, die in äquivalenter Weise mit IA-2 bindefähig sind wie die Antikörper, die von der Zellinie IA-2, 96-3-1, Hinterlegungsnummer DSM ACC2365, produziert werden.

7. Zellinie IA-2, 96-3-1 mit der Hinterlegungsnummer DSM ACC2365.

8. Verfahren zur Herstellung von humanen monoklonalen Antikörper nach einem der Ansprüche 1 bis 6 enthaltend die Schritte Immortalisieren von humanen Lymphozyten von Prädiabetikern oder Diabetikern mit hohen Serumantikörper-Titern gegen IA-2, Kultivierung der immortalisierten Lymphozyten mit Wachstumsfaktoren unter gleichzeitiger Entfernung von inhibitorischen Faktoren, Nachweis der IA-2-spezifischen humanen monoklonalen Antikörper im Kulturüberstand, Klonierung der humanen immortalisierten Zellinie, die diesen Antikörper produziert, in Anwesenheit von Feederzellen, die keine cytotoxischen T-Lymphozyten enthalten, Vermehrung dieser immortalisierten Zelle gegebenenfalls unter Zusatz von Wachstumsfaktoren, und Isolierung des von diesem Klon produzierten monoklonalen Antikörpers.

9. Verwendung eines humanen monoklonalen Antikörpers nach einem der Ansprüche 1 bis 6 als Standard in einem Verfahren zur Bestimmung von Antikörpern gegen ein Inselzellantigen.

10. Verwendung eines humanen monoklonalen Antikörpers nach einem der Ansprüche 1 bis 6 als Rezeptor in einem Verfahren zur Bestimmung von Antikörpern gegen ein Inselzellantigen.

11. Verwendung eines humanen monoklonalen Antikörpers nach einem der Ansprüche 1 bis 6 zur Gewinnung des Inselzellantigens IA-2.

12. Verfahren zum Nachweis von Antikörpern gegen das Inselzellantigens IA-2 in einer Probe, **dadurch gekennzeichnet, daß** als Standard ein monoklonaler Antikörper gemäß einem der Ansprüche 1 bis 6 eingesetzt wird.

13. Anti-idiotypischer Antikörper, der gegen Antikörper, die mit dem Inselzellantigen IA-2 reagieren, gerichtet ist, erhältlich durch die Immunisierung mit einem Antikörper gemäß einem der Ansprüche 1 bis 6, Immortalisierung der Milzzellen der immunisierten Tiere, Klonierung von solchen immortalisierten Zellen, die Antikörper produzieren, die an Antikörper gegen IA-2 binden und Isolierung der von diesen Klonen produzierten Antikörper nach bekannten Verfahren.

14. Verfahren zum Nachweis des Inselzellantigens IA-2 in einer Probe, **dadurch gekennZeichnet, daß** als Bindepartner mindestens ein monoklonaler Antikörper gemäß den Ansprüchen 1 bis 6 eingesetzt wird.

## Claims

1. Human monoclonal antibodies which can be obtained by the process steps of immortalizing human lymphocytes from prediabetics or diabetics with high serum antibody titres to IA-2, culturing the immortalized lymphocytes with growth factors while simultaneously removing inhibitory factors, detecting the IA-2-specific human monoclonal antibodies in the culture supernatant, cloning the human immortalized cell line which produces this antibody in the presence of feeder cells which contain no cytotoxic T lymphocytes, proliferating this immortalized cell optionally with the addition of growth factors and isolating the monoclonal antibody produced by this clone.

2. Human monoclonal antibodies according to claim 1, wherein they react specifically with IA-2ic, the cytoplasmic part of IA-2.

3. Human monoclonal antibodies according to one of the claims 1 to 2, wherein they belong to the IgG class.

4. Human monoclonal antibodies according to claim 3, wherein they belong to the subclass IgG1.

5. Human monoclonal antibodies according to claims 1 to 4, wherein they can be obtained from the cell line IA-2, 96-3-1 with the depository number DSM ACC2365.

6. Human monoclonal antibodies according to claims 1 to 5, which can bind to IA-2 in an equivalent manner to the antibodies that are produced by the cell line IA-2, 96-3-1, depository number DSM ACC2365.

7. Cell line IA-2, 96-3-1 with the depository number DSM ACC2365.

8. Process for producing human monoclonal antibodies according to one of the claims 1 to 6 comprising the steps: immortalizing human lymphocytes from prediabetics or diabetics with high serum antibody titres to IA-2, culturing the immortalized lymphocytes with growth factors while simultaneously removing inhibitory factors, detecting the IA-2-specific human monoclonal antibodies in the culture supernatant, cloning the human immortalized cell line which produces this antibody in the presence of feeder cells which contain no cytotoxic T lymphocytes, proliferating this immortalized cell optionally with the addition of growth factors and isolating the monoclonal antibody produced by this clone.

9. Use of a human monoclonal antibody according to one of the claims 1 to 6 as a standard in a method for determining antibodies to an islet cell antigen.

10. Use of a human monoclonal antibody according to one of the claims 1 to 6 as a receptor in a method for determining antibodies to an islet cell antigen.

11. Use of a human monoclonal antibody according to one of the claims 1 to 6 for isolating the islet cell antigen IA-2.

12. Method for detecting antibodies to the islet cell antigen IA-2 in a sample, wherein a monoclonal antibody according to one of the claims 1 to 6 is used as a standard.

13. Anti-idiotypic antibody which is directed against antibodies that react with the islet cell antigen IA-2 which can be obtained by immunization with an antibody according to one of the claims 1 to 6, immortalizing the spleen cells of the immunized animals, cloning those immortalized cells which produce antibodies that bind to antibodies against IA-2 and isolating the antibodies produced by these clones by known methods.

14. Method for detecting the islet cell antigen IA-2 in a sample, wherein at least one monoclonal antibody according to one of the claims 1 to 6 is used as the binding partner.

## Revendications

1. Anticorps monoclonaux humains que l'on obtient en passant par les étapes opératoires : immortaliser des lymphocytes humains de prédiabétiques ou de diabétiques possédant des titres d'anticorps sériques élevés contre IA-2, cultiver les lymphocytes immortalisés avec des facteurs de croissance tout en procédant à une élimination simultanée des facteurs inhibiteurs, déceler les anticorps monoclonaux humains manifestant une spécificité pour IA-2 dans le produit surnageant, cloner la lignée cellulaire immortalisée humaine qui produit ces anticorps en présence de cellules nourricières qui ne contiennent aucun lymphocyte T cytotoxique, multiplier ces cellules immortalisées, le cas échéant en ajoutant des facteurs de croissance, et isoler les anticorps monoclonaux produits par ce clone.

2. Anticorps monoclonaux humains selon la revendication 1, **caractérisés en ce qu'**ils réagissent de manière spécifique avec IA-2ic, la partie cytoplasmique de IA-2.

3. Anticorps monoclonaux humains selon l'une quelconque des revendications 1 à 2, **caractérisés en ce qu'**ils font partie de la classe des IgG.

4. Anticorps monoclonaux humains selon la revendication 3, en ce qu'ils font partie de la sous-classe des IgG1.

5. Anticorps monoclonaux humains selon l'une quelconque des revendications 1 à 4, que l'on obtient à partir de la lignée cellulaire IA-2, 96-3-1 portant le numéro de dépôt DSM ACC2365.

6. Anticorps monoclonaux humains selon les revendications 1 à 5, qui manifestent une aptitude à la liaison à IA-2 équivalente à celle des anticorps que l'on obtient à partir de la lignée cellulaire IA-2, 96-3-1, numéro de dépôt DSM ACC2365.

7. Lignée cellulaire IA-2, 96-3-1, portant le numéro de dépôt DSM ACC2365.

8. Procédé pour la préparation d'anticorps monoclonaux humains selon l'une quelconque des revendications 1 à 6, comprenant les étapes : immortaliser des lymphocytes humains de prédiabétiques ou de diabétiques possédant des titres d'anticorps sériques élevés contre IA-2, cultiver les lymphocytes immortalisés avec des facteurs de croissance tout en procédant à une élimination simultanée des facteurs inhibiteurs, déceler les anticorps monoclonaux humains manifestant une spécificité pour IA-2 dans le produit surnageant, cloner la lignée cellulaire immortalisée humaine qui produit ces anticorps en présence de cellules nourricières qui ne contiennent aucun lymphocyte T cytotoxique, multiplier ces cellules immortalisées, le cas échéant en ajoutant des facteurs de croissance, et isoler les anticorps monoclonaux produits par ce clone.

9. Utilisation d'un anticorps monoclonal humain selon l'une quelconque des revendications 1 à 6, à titre de référence dans un procédé pour la détermination d'anticorps contre un antigène des cellules d'îlots de Langerhans.

10. Utilisation d'un anticorps monoclonal humain selon l'une quelconque des revendications 1 à 6, à titre de récepteur dans un procédé pour la détermination d'anticorps contre un antigène des cellules d'îlots de Langerhans.

11. Utilisation d'un anticorps monoclonal humain selon l'une quelconque des revendications 1 à 6, pour l'obtention de l'antigène IA-2 des cellules d'îlots de Langerhans.

12. Procédé pour le décèlement d'anticorps contre l'antigène IA-2 des cellules d'îlots de Langerhans dans un échantillon, **caractérisé en ce qu'**on met en oeuvre, à titre de référence, un anticorps monoclonal selon l'une quelconque des revendications 1 à 6.

13. Anticorps anti-idiotype qui est dirigé contre des anticorps qui réagissent avec l'antigène IA-2 des cellules d'îlots de Langerhans, que l'on obtient par immunisation avec un anticorps selon l'une quelconque des revendications 1 à 6, immortalisation des cellules spléniques des animaux immunisés, clonage de cellules immortalisées de ce type, qui produisent des anticorps qui se lient à des anticorps dirigés contre IA-2 et isolation des anticorps produits par ces clones conformément à des procédés connus.

14. Procédé pour le décèlement de l'antigène IA-2 d'îlots de Langerhans dans un échantillon, **caractérisé en ce qu'**on met en oeuvre, à titre de partenaire de liaison, au moins un anticorps monoclonal selon les revendications 1 à 6.
